# EUROPEAN PATENT APPLICATION

(11) **EP 2 511 368 A1**
(43) Date of publication of application: **17.10.2012**
(21) Application number: 09851471.4
(22) Date of filing: 20.11.2009
(51) Int. Cl.: C12N 7/00, A61K 35/76, A61K 39/155, A61K 39/395, A61P 31/14, C12N 15/09

(54) **RECOMBINANT MEASLES VIRUS USEFUL AS BIVALENT VACCINE AGAINST MEASLES AND NIPAH VIRUS INFECTION**

(71) Applicant: aRigen Pharmaceuticals, Inc., Minato-ku Tokyo 107-0052 (JP)
(72) Inventor: KAI. Chieko, Tokyo 153-0041 (JP); YONEDA, Misako, Tokyo 134-0081 (JP)
(74) Representative: Faivre Petit, Frédérique
(86) International application number: PCT/JP2009/069692
(87) International publication number: WO 2011/061849

(57) **Abstract**

The object of the present invention is to provide a vaccine which is safe and effective against Nipah virus infection and a vector which is used in the manufacture of this vaccine and to provide a bivalent vaccine which exhibits an excellent preventive effect against measles virus and Nipah virus infection and which eliminates complexity at the time of inoculation. The present invention provides a recombinant measles virus in which is inserted a gene which encodes a protein involved in preventing Nipah infection into the measles virus genome. The protein involved in preventing Nipah virus infection is preferably G protein or F protein which is a membrane protein. The present invention also relates to a bivalent vaccine against measles and Nipah virus infection which contains the recombinant measles virus, and an antiserum obtained from body fluid taken from an animal infected with the recombinant measles virus. It also provides a method of manufacturing a vaccine against Nipah virus infection which is characterized by using a measles virus as a vaccine vector in the manufacture of a vaccine against Nipah virus infection.

## Description

### Technical Field

This invention relates to a recombinant measles virus having a vaccine action against measles and Nipah virus infections, a vaccine containing the virus, and an antiserum obtained using the virus.

### Background Art

Nipah virus infection is an emerging virus infection which first appeared from 1998 to 1999 in Malaysia. 265 patients exhibited severe respiratory symptoms, encephalitis, and other symptoms, and 107 fatalities occurred. It was discovered that the first epidemic occurred in pig-raising areas and that infection spread from pigs to humans. The natural host is the fruit bat. There has not been a recurrence of the disease in Malaysia since that time, but from 2003 to 2004, an epidemic occurred in Bangladesh with a mortality reaching 70% and above, and more than 50 people died. In Bangladesh, Nipah virus infection still sporadically occurs, and each year a certain number of patients die. In Bangladesh, pigs are not involved in its spread, and it is strongly suggested that humans are directly infected from fruit bats, which are its natural host, or that the virus is spread from human to human.

The principal clinical symptom in humans is encephalitis. The incubation period is 4 - 18 days, after which symptoms begin with fever followed by headache and sleepiness. Some patients complain of neck stiffness, uneasiness, paralysis, nausea, and dizziness. Symptoms such as loss of sense of direction, confusion, abnormal behavior, and memory loss may also occur, and in severe cases, coma and death occur after 3 to 30 days. If recovery takes place, it takes from 3 days to around 2 weeks. Respiratory ailments rarely occur.

Nipah virus is a virus belonging to the genus Henipavirus of the family Paramyxoviridae. The most closely related Hendra virus is a virus which causes an emerging virus infection which appeared in Australia in 1994. Horses died from hemorrhagic pneumonia, humans were infected from horses, and a total of two persons died from acute respiratory symptoms and encephalitis. Nipah virus and Henipah virus both infect host cells with Ephrin-B2, which is a transmembrane protein having a cell signal transmitting domain, as a receptor. Ephrin-B2 is known to be expressed in vascular endothelial cells and cerebral nerve cells, and this is one explanation for a broad host range of Nipah virus and why it can cause systemic infections.

Treatment is conducted by normal symptomatic therapy of the viral disease. At present, an effective treatment method does not exist. The anti-viral drug Ribavirin is also used, but its effectiveness is unclear. If symptoms become serious, the mortality rate is high, and even after recovery, there are many cases in which there are residual neurological symptoms. A certain effect has been exhibited in animal experiments by immunity using vaccinia virus which expresses the F protein and G protein of Nipah virus as a vaccine. However, the safety of this vaccine has not been ascertained with respect to humans having a immune suppression such as AIDS patients, so a recombinant virus vaccine using vaccinia virus has not been put to practical use.

Until recently, measles was the best known of common infant diseases. Clinical characteristics include premonitory symptoms such as fever, head cold, and conjunctivitis, after which a rash appears on the head and spreads to the chest, torso, and limbs. Measles often causes complications such as otitis media, croup, bronchitis, and bronchopneumonia. When undernourished infants die of measles, the cause of death is usually bacterial pneumonia. However, the most dangerous complication of measles is acute infectious encephalopathy, which occurs at a rate of 1 case per 1000 cases of measles and has a mortality rate of as high as approximately 15%. Even if death is avoided, in many cases, damage to the nervous system remains for life.

In countries where preventive inoculation is not carried out to a great extent, major measles epidemics occur every two or three years, and much occurrence is experienced primarily from late fall to spring. A measles vaccine was approved in the United States in 1963, but prior to that time, major epidemics occurred every two or three years, and it has been reported that in years with a major epidemic, there were 500,000 cases of measles per year and approximately 500 deaths from measles per year. From 1963 onwards, there was a sharp decrease in reports of measles patients, major epidemics every two or three years were no longer observed, and in 1983, there were only 1497 reported cases of measles.

However, in developing countries, there is a high mortality rate due to infantile measles within one year after birth, and the World Health Organization (WHO) has made preventive inoculation a priority and is carrying this out as a portion of expanded preventive inoculation. In these regions, the decrease in antibodies from the mother's body is faster than in developed countries, and infants rapidly become receptive to measles. As a result, the death of undernourished infants due to measles is a major cause of infant deaths.

Measles is caused by the measles virus, which belongs to the genus Morbillivirus of the family Paramyxoviridae. Virus particles have a polymorphic elliptical structure with a diameter of 100 - 250 nm. The internal capsid contains the spiral-shaped negative RNA genome, and the external envelope comprises a matrix protein (M), and short surface glycoprotein spikes of hemagglutinin (H) protein and fusion (F) protein. The H and F proteins are necessary for cell adsorption and cell fusion of the virus, and the M protein is necessary for assembly of the virus. The virus proteins include six types of structural proteins and one type of non-structural protein.

An attenuated live vaccine which comprises measles viruses attenuated by serial subculture on cells is used as a vaccine against measles. The immune effect thereof continues for long periods.

However, up to now, there has been no idea of using a measles virus as a vector for a vaccine against Nipah virus infection.

### Disclosure of Invention

### Problem Which the Invention is to Solve

As stated above, an effective vaccine against Nipah virus infection has thus far not been put to actual use. Accordingly, the object of the present invention is to provide a vaccine which is safe and effective against Nipah virus infections and a vector for use in manufacturing such a vaccine. A further object of the present invention is to provide a bivalent vaccine which exhibits an excellent preventative effect against measles virus and Nipah virus infections and which eliminates complexities at the time of inoculation.

### Means for Solving the Problem

During investigations with the purpose of developing an effective Nipah virus vaccine, the present inventors focused on the manufacture of a vaccine using a virus vaccine vector, and in this process, they conceived of using a measles virus as a vector. The safety and effectiveness of a measles virus as a live vaccine has been established, so the present inventors thought that it could be used as a virus vaccine vector from the standpoints of inducing an effective immune reaction and imparting lifetime immunity. Therefore, they attempted to insert a gene encoding an antigen involved in the prevention of Nipah virus infection into the measles virus to produce a recombinant measles virus. As a result of diligent investigation, the present inventors found that a recombinant measles virus which is obtained in this manner stably expresses the gene of an antigen involved in preventing Nipah virus infection, which is an inserted foreign gene inside an infected cell, and that it can be used as a virus vector for a multivalent vaccine. Namely, they were able to obtain a recombinant measles virus which is useful as a vaccine against measles and Nipah virus infections.

Accordingly, the gist of the present invention is a recombinant measles virus having a gene which encodes a protein involved in preventing Nipah virus infection inserted into the measles virus genome.

In addition, the present invention is a recombinant measles virus having infectivity, which can express in an infected cell a protein which produces a defense against Nipah virus infection after inoculation with the virus.

The present invention also relates to the above-described recombinant measles virus characterized in that at least one gene in the measles virus genome and particularly a measles virus functional protein gene is modified.

The above-described recombinant measles virus may contain a foreign gene other than a gene which encodes a protein involved in preventing Nipah virus infection such as a gene of an antigen recognition site for a monoclonal antibody against a cancer-specific marker molecule.

The present invention also relates to RNA contained in the above-described recombinant measles virus and DNA which comprises a template cDNA which can transcribe this recombinant measles virus genome RNA. This DNA is preferably in the form of a plasmid.

The present invention also relates to a bivalent vaccine against measles and Nipah virus infection comprising the above-described recombinant measles virus. It relates to antiserum obtained from a body fluid collected from an animal infected with the above-described recombinant measles virus. The present invention also provides a method of manufacturing a vaccine against Nipah virus infection characterized by using a measles virus as a vaccine vector.

### Effects of the Invention

As explained above, the present invention can provide a vaccine against Nipah virus infection which thus far has not been put to practical use, and defense against measles and Nipah virus infection becomes possible by inoculation with a single recombinant measles vaccine. In addition, diagnosis and treatment of Nipah virus infection by an antiserum against Nipah virus infection are made possible.

### Brief Explanation of the Drawings

Figure 1 - This is a figure schematically showing the construction of pMV-HL (7+).
Figure 2 - This is a figure showing expression of NiVG in a recombinant measles virus (MV-NiVG)-infected cell.

### Embodiments of the Invention

Below, a recombinant measles virus and a vaccine containing it according to the present invention will be explained in detail.

In the present invention, the manufacture of a vaccine against Nipah virus infection is characterized in that a measles virus is used as a vaccine vector. Namely, a recombinant measles virus having a gene which encodes a protein involved in defense against Nipah virus infection inserted into the measles virus genome is prepared and used as a vaccine.

Preparation of a recombinant measles virus according to the present invention can utilize a reverse genetic system which is used for reconstruction of a canine distemper virus described in JP 2001-275684, for example. Gene manipulation such as modification of a virus genome and introduction of a foreign gene is carried out at the level of DNA, so in gene manipulation of a measles virus which belongs to a (-) strand RNA virus, it is necessary to obtain cDNA of a virus genome. Namely, it is necessary to reconstitute a recombinant measles virus particle having infectivity through cDNA. More specifically, cDNA of measles virus genome RNA is obtained, and the target foreign gene is incorporated therein to obtain a recombinant cDNA. By introducing this recombinant cDNA into a cell which expresses genes related to transcription and replication together with a unit which can transcribe into RNA using this cDNA as a template, it is possible to reconstitute a measles virus particle.

Any measles virus which can induce a high titer of a neutralizing antibody against a field measles virus is suitable as a measles virus for use in the preparation of a recombinant measles virus, examples of which are HL strain and ICB strain. It is also possible to use a vaccine strain such as the Edmonston strain or AIC strain which has been modified by genetic engineering so as to induce production of corresponding neutralizing antibodies.

A gene of a Nipah virus which is inserted as a foreign gene into the measles virus genome may be any gene which encodes a protein which brings about defense against Nipah virus infection such as a F gene or a G gene which encodes a virus membrane protein.

The base sequences of all the genes of the Nipah virus have been determined. For example, the base sequences of the F gene and the G gene are recorded as AF2123021 in the GenBank database. Therefore, using a set of primers which is designed based on the known sequences, it is possible to obtain cDNA of the F gene or the G gene using RNA which is extracted from a cell infected by Nipah virus.

At its ends, the genome of a measles virus has a leader sequence which participates in virus replication and a trailer sequence, and between these it has N, P, M, F, H, and L genes which encode constitutive proteins of the virus. The N protein is successively connected to virus RNA from the 3' end and packages it. It has been found that the P protein, V protein, and C protein are produced from the P gene, and that the P protein participates in transcription and replication of viruses as a small subunit of RNA polymerase. L protein functions as a large subunit of RNA polymerase. M protein supports the virus particle structure from the interior, and F protein and H protein relate to invasion of host cells.

In order to construct a recombinant measles virus according to the present invention, first, a genome RNA is prepared from a measles virus like that described above, and its cDNA is prepared. The cDNA is linked downstream of a specific promoter. Depending upon the orientation of the cDNA, genome RNA or cRNA is transcribed. cDNA(s) of the above-described gene(s) of a Nipah virus is (are) inserted into the cDNA of the measles virus by gene manipulation to construct a recombinant cDNA.

In the construction of a recombinant measles virus according to the present invention, there is no particular limitation on the site on a measles virus genome where a gene which encodes an antigen involved in protection against Nipah virus infection is inserted, and it can be inserted between any two of the N, P, M, F, H, and L genes or upstream of the N gene.

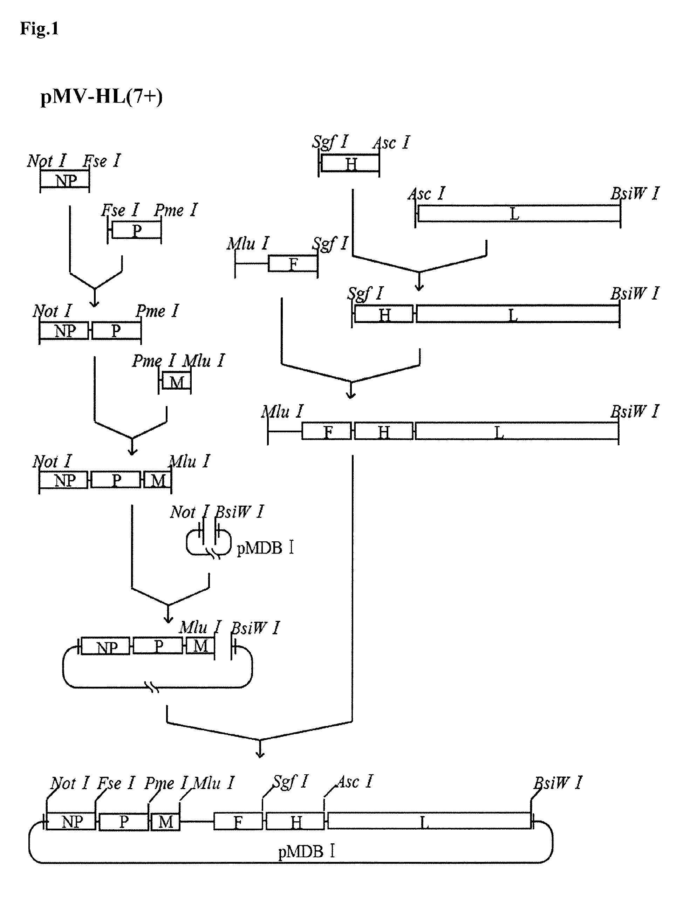

When preparing cDNA of a measles virus genome, if a suitable restriction enzyme recognition sequence is placed between every two adjoining genes of the N, P, M, F, H, and L genes which encode a protein which constitutes the virus, the target foreign gene can be easily inserted, and a location enabling optimal expression of foreign proteins can be selected. A concrete example in which restriction enzyme recognition sequences are disposed between the genes is the plasmid pMV (7+) used in Example 1, for example (see Figure 1).

As long as a recombinant measles virus according to the present invention is effective at preventing Nipah virus infection and maintains infectivity, any other foreign gene can be inserted at an arbitrary site of RNA contained in the recombinant, or any gene in the genome can be missing or altered. Examples of other foreign genes which can be inserted are genes which encode various proteins which cause pathogenicity of viruses, bacteria, or parasites, genes which encode various cytokines, genes which encode various peptide hormones, and genes which encode antigen recognition sites in antibody molecules, with each of the above examples being genes which can be expressed in the host. For example, there are antigen recognition site genes for monoclonal antibodies against influenza virus, mumps virus, HIV, dengue fever virus, diphtheria, leishmaniasis, and cancer-specific marker molecules. The amount of expression of the inserted foreign gene can be adjusted by the site of gene insertion or by the RNA base sequence before and after the gene. For example, a gene which participates in immunogenicity may be inactivated. Alternatively, a portion of a gene which participates in RNA replication of a measles virus may be altered in order to increase the transcription efficiency or replication efficiency of RNA. Specifically, an intervening sequence or the leader portion may be modified, for example.

A recombinant virus can be formed by introducing cDNA of a measles virus genome on which the above-described gene manipulation was carried out together with a unit which can transcribe into RNA using this DNA as a template inside a cell into a host which expresses all enzymes for transcription and replication of a measles virus or a closely related virus. An example of a unit which can transcribe into RNA is DNA which expresses DNA-dependent RNA polymerase which acts on a specified promoter, downstream of which cDNA which has undergone the above-described gene manipulation is connected. Specific examples of this unit are recombinant vaccinia virus which expresses T7RNA polymerase or cultured cells into which T7RNA polymerase gene has been artificially incorporated.

The host into which the cDNA is introduced together with this unit can be any host which expresses all enzymes for transcription and replication of measles virus or a closely related virus, namely, a host which simultaneously expresses N protein, P protein, and L protein, or proteins having equivalent activity to these proteins. For example, a cell having genes which encode these proteins on its chromosome can be used, or a suitable cell into which plasmids having genes which encode each of N protein, P protein, and L protein can be used. 293 cells, B95a cells, or the like into which suitable plasmids having N gene, P gene, and L gene have been introduced are preferred.

A recombinant measles virus according to the present invention which is obtained in the above-described manner contains a gene which encodes a protein which produces a defense against Nipah virus infection. As proved by the below-described examples, after inoculation of the recombinant virus, this gene expresses a protein which brings about a defense against Nipah virus infection inside an infected cell. Therefore, it exhibits the effect of preventing proliferation of Nipah virus. Moreover, this recombinant virus maintains its function as a measles virus, so it is also effective as a vaccine against measles.

A vaccine according to the present invention can be manufactured by methods ordinarily used in this field, including, if necessary, mixing a recombinant measles virus according to the present invention with a pharmacologically acceptable carrier or suitable additive. A pharmacologically acceptable carrier is a diluent, an excipient, a binder, a solvent, or the like which does not cause a harmful physiological reaction in the subject to which it is administered and which does not produce a harmful interaction with other components of the vaccine composition. For example, water, physiological saline, and various buffering agents can be used. Examples of additives which can be used include adjuvants, stabilizers, isotonizing agents, buffers, solubilizers, suspending agents, preservatives, cryoprotective agents, freezing protective agents, freeze drying protective agents, and bacteriostats.

The vaccine may be in liquid, frozen, or freeze dried form. A liquid vaccine can be manufactured by collecting a cultured liquid which is obtained by culturing in a suitable medium or cultured cells or the like, adding an additive such as a stabilizer, and sealing the vaccine in a small bottle or ampoule. A frozen vaccine is obtained by gradually lowering the temperature and freezing after placing the vaccine into a container, with a cryoprotective agent or a freezing protective agent being added. A freeze dried vaccine is obtained by freezing the container into which the vaccine was dispensed in a freeze dryer and then performing vacuum drying and then sealing the container either as is or after charging nitrogen gas into the container. A liquid vaccine can be used as is or after being diluted with physiological saline or the like. When a frozen or freeze dried vaccine is used, a dissolving liquid for dissolving the vaccine is used. All types of buffers or physiological saline can be used as the dissolving liquid.

As an adjuvant for increasing immunogenicity, ones customarily used in this field can be employed, including cells such as BCG and Propionibacterium acnes, bacterial components such as cell walls and trehalose dimycolate (TDM), lipopolysaccharide (LPS) or lipid A fractions which are endotoxins of gram negative bacteria, beta-glucan, N-acetyl muramyl dipeptide (MDP), bestatin, synthetic compounds such as levanisole, thymus hormones, thymic factors, proteins derived from components of organisms such as tuftsin, peptide products, Freund's incomplete adjuvant, and Freund's complete adjuvant.

The vaccine can be administered by subcutaneous administration, intramuscular administration, intravenous administration, or the like. The dosage depends upon the age, weight, and sex of the subject and the method of administration and is not particularly limited, but it is normally preferably in the range of 10⁴ - 10⁷ TCID₅₀ per administration, and it is particularly preferable for it to be at least 10⁵ TCID₅₀. Administration is preferably carried out in the same manner as for a measles vaccine.

It is also possible to infect an animal with the recombinant measles virus, to obtain an antiserum or the like from a body fluid of the animal, and to use the antiserum for treatment and diagnosis.

The present invention will be explained more concretely by the following examples, but the present invention is not limited by these examples.

### Example 1

Constructing a recombinant measles virus having a Nipah virus G protein gene (MV-NiVG)

pMV(7+) which was constructed based on the entire gene sequence of the genome of a field HL strain of measles virus and by artificially disposing a restriction enzyme recognition sequence at both ends of each of 6 types of genes which encode constitutive proteins of the virus was used as an infectious cDNA clone necessary for preparing recombinant MV (Figure 1).

The cDNA of the gene of the G protein, which is a membrane protein of the Nipah virus, was obtained by RT-PCR using overall RNA extracted from a Nipah virus-infected vero cell. NiV-G cDNA was reamplified by a pair of primers to which was added Fse I restriction enzyme recognition sequence, it was cloned in a plasmid vector, and the base sequence was inspected. The NiV-G cDNA which was obtained by digesting this plasmid with Fse I was inserted at the Fse I site between the N gene and the P gene of pMV (7+) to obtain infectious cDNA clone pMV-NiVG which is used to construct a recombinant measles virus having G gene of a Nipah virus.

The reconstitution of recombinant measles virus was carried out as follows.

293 cells which had been trypsinized in a usual manner and 2 ml of DMEM medium containing 5% fetal bovine serum were placed into a 6-well plate (1,000,000 cells/well) and were incubated for 24 hours under 5% CO₂ at 37°C. After the culture liquid was removed, a suspension of recombinant vaccinia virus MVA-T7 which can express T7 RNA polymerase in 0.2 ml of PBS was added to each well so that the multiplicity of infection (moi) became 2. The plate was shaken every 10 minutes so that the virus liquid spread over the entire well, and infection was carried out for 1 hour. After 1 hour, the virus liquid was removed, 2 ml of medium were added to each well, and 100 µl of cDNA solution were added dropwise. The cDNA solution was prepared in the following manner.

1 µg, 1 µg, and 0.1 µ, respectively, of plasmids pGEM-NP, pGEM-P, and pGEM-L necessary for replication of measles virus were collected with a 1.5-ml sampling tube. 1 µg of pMV(7+)-NiVG and sterilized distilled water were added to the tube to prepare 10 µl of nucleic acid solution. 0.08 ml of DMEM medium were prepared in a separate sampling tube, 10 µl of Fugene 6 (Roche Diagnostics) were added dropwise to the DMEM medium, and the mixture was left standing for 5 minutes in this state at room temperature. This mixture was blended with the nucleic acid solution and left standing for at least 15 minutes at room temperature to obtain the cDNA solution.

A plate containing the above-described wells was incubated for 3 days under 5% CO₂ at 37°C. On the third day, the medium was removed, and B95a cells which were suspended in a RPMI-1640 medium containing 5% fetal bovine serum were overlaid in an amount of 1,000,000 cells per well on the 293 cells. This plate was further incubated under 5% CO₂ at 37°C until a cytopathogenic effect (CPE) was observed.

The formation of a recombinant measles virus (MV-NiVG) was confirmed by RT-PCR and sequencing.

### Example 2

Confirmation of expression of Nipah virus G protein in recombinant measles virus (MV-NiVG)-infected cells

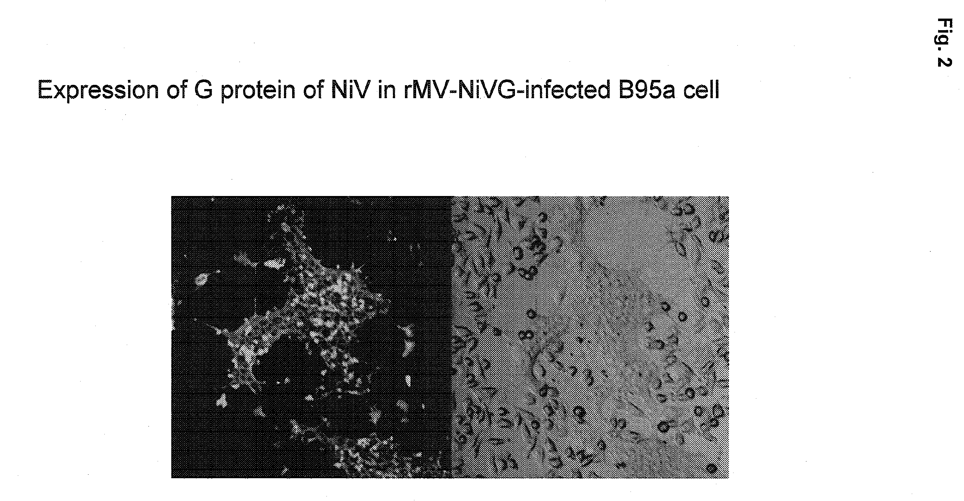

B95a cells were infected with MV-NiVG, after 48 hours the cells were fixed in 4% paraformaldehyde and permeated with 0.2% Triton X-100. Anti-NiVG antibodies (rabbit serum) diluted 1000 times were added and reacted for 1 hour at room temperature. The NiVG antibodies were removed, washing was performed 3 times with PBS, FITC-labeled anti-rabbit IgG antibodies diluted 2000 times were added, and the mixture was reacted for 30 minutes at room temperature. The anti-rabbit IgG antibodies were removed, and washing with PBS was performed 5 times, after which the infected cells were observed using a confocal laser microscope. As a result, fluorescence of FITC was observed only in MV-NiVG infected cells, and the expression of NiVG antigens was ascertained (Figure 2).

## Claims

1. A recombinant measles virus having a gene which encodes a protein involved in preventing Nipah virus infection inserted into the measles virus genome.

2. A recombinant measles virus having infectivity which can express a protein which produces the effect of preventing Nipah virus infection after inoculation with the virus in an infected cell.

3. A recombinant measles virus as set forth in claim 1 or claim 2 **characterized in that** at least one measles virus genome gene is modified.

4. A recombinant measles virus as set forth in any of claims 1 - 3 **characterized by** further containing a foreign gene other than a gene which encodes a protein involved in preventing Nipah virus infection.

5. RNA contained in a recombinant measles virus as set forth in any of claims 1 - 4.

6. DNA comprising a template cDNA which can transcribe a recombinant measles virus genome RNA as set forth in claim 5.

7. DNA as set forth in claim 6 which is in the form of a plasmid.

8. A bivalent vaccine against measles and Nipah virus infection containing a recombinant measles virus as set forth in any of claims 1 - 4 and a pharmacologically acceptable carrier.

9. An antiserum obtained from a body fluid taken from an animal infected with a recombinant measles virus as set forth in any of claims 1 - 4.

10. A method of manufacturing a vaccine against Nipah virus infection, **characterized by** using a measles virus as a vaccine vector.
